# EUROPEAN PATENT SPECIFICATION

(11) **EP 0 810 224 B1**
(45) Date of publication and mention of the grant of the patent: **01.08.2001**
(21) Application number: 97108713.5
(22) Date of filing: 30.05.1997
(51) Int. Cl.: C07D 405/12

(54) **Method of producing amorphous paroxetine hydrochloride**
Verfahren zur Herstellung amorphen Paroxetin-Hydrochlorids
Procédé de préparation de l'hydrochlorure de paroxétine amorphe

(30) Priority: 30.05.1996 JP 13719296
(43) Date of publication of application: 03.12.1997
(60) Divisional application: 00125372.3
(73) Proprietor: ASAHI GLASS COMPANY LTD., Tokyo 100-8405 (JP)
(72) Inventor: Wang, Shu-zhong, Asahi Glass Company Ltd., Yokohama-shi, Kanagawa (JP); Okazoe, Takashi, Asahi Glass Company Ltd., Yokohama-shi, Kanagawa (JP); Matsumura, Yasushi, Asahi Glass Company Ltd., Yokohama-shi, Kanagawa (JP)
(74) Representative: Müller-Boré & Partner Patentanwälte

(56) References cited:
- EP-A- 0 223 403
- EP-A- 0 542 355
- WO-A-95/15155
- US-A- 4 007 196

## Description

The present invention relates to a method of producing paroxetine hydrochloride which has an inhibitory action on 5-hydroxytryptamine (5-HT) and is useful as a therapeutic agent for various diseases such as depression and Parkinson's diseases.

Paroxetine hydrochloride, i.e. (3S,4R)-3-[5-(1,3-dioxaindanyl)oxymethyl]-4-(p-fluorophenyl)piperidine hydrochloride, has been obtainable in the crystalline form, and hemihydrated paroxetine hydrochloride is also known in the crystalline form (U.S.P No. 4,007,196, and Japanese Examined Patent Publication JP-B-6-47587). However, because of their crystallinity, they are medicines which are slowly absorbed into the body.

To solve the problem, the present invention provides a method of producing amorphous paroxetine hydrochloride which is generally easy to absorb.

Namely, the present invention provides a method of producing amorphous paroxetine hydrochloride, which comprises converting paroxetine or its lower alkanoic acid salt into paroxetine hydrochloride, and then spray drying a solution of the hydrochloride.

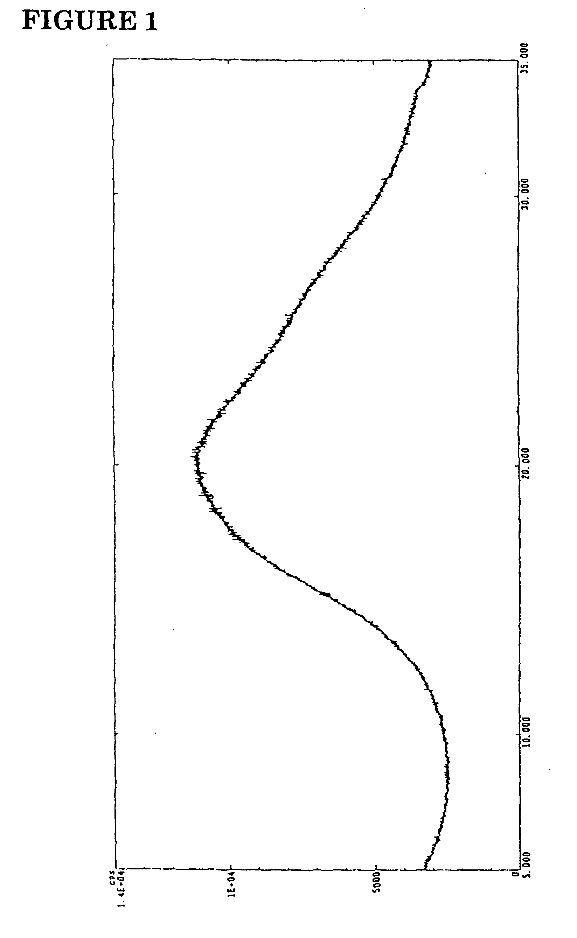

Figure 1 is the X-ray powder diffraction pattern of the amorphous paroxetine hydrochloride.

Paroxetine is obtainable by the method disclosed in U.S.P. 4,007,196 and the like. A lower alkanoic acid salt of paroxetine can be obtained easily by mixing paroxetine with the corresponding lower alkanoic acid.

In the present invention, "lower" means a carbon number of at most 6. As the lower alkanoic acid, an alkanoic acid having a carbon number of at most 4 is preferred, and the most preferred is acetic acid.

In the method of the present invention, it is preferred to dissolve paroxetine or its alkanoic acid salt in an appropriate solvent, and then add a solution of hydrogen chloride in an appropriate solvent or directly introduce gaseous hydrogen chloride to the resulting paroxetine solution to prepare a solution of paroxetine hydrochloride. The amount of hydrogen chloride to be used is preferably at least equivalent to the used paroxetine or its salt. The reaction temperature is preferably from -20 to +50°C, more preferably from -10 to +30°C.

The solvent to be used is in general a solvent which dissolves amines and their salts, and preferably a saturated alcohol having a boiling point of at most 170°C, or water. Particularly preferred are lower saturated alcohols such as methanol, ethanol, 1-propanol, 2-propanol, 1-butanol, 2-butanol, 2-methyl-1-propanol and 2-methyl-2-propanol. The most preferred solvent is ethanol.

Then, the resulting solution of paroxetine hydrochloride is concentrated in vacuo to remove the substances other than paroxetine hydrochloride in the system upon evaporation. When a saturated alcohol is used as the solvent, the lower alkanoic acid in the system is removed in the form of an ester.

The resulting residue is already amorphous at this time, but an amorphous substance like this is usually so hygroscopic that it is usually hard to handle. Powdery amorphous paroxetine hydrochloride which is easy to handle can be obtained by dissolving the residue in an appropriate solvent and spray drying the paroxetine hydrochloride solution.

The solvent to be used in this step may be any solvent which dissolves paroxetine hydrochloride, and, preferably has a boiling point at most 120°C. For example, toluene, tetrahydrofuran, 1,4-dioxane, 1,2-dimethoxyethane, methanol, ethanol, 2-propanol, 2-methyl-2-propanol, water or a mixture thereof may be mentioned. Particularly preferred are lower alcohols, and the most preferred solvent is ethanol.

When a lower saturated alcohol such as ethanol is used as the solvent in the above step to prepare a paroxetine hydrochloride solution from paroxetine or its alkanoic acid salt, the resulting paroxetine hydrochloride solution can be directly spray dried without concentration to obtain amorphous paroxetine hydrochloride. When a lower alkanoic acid salt of paroxetine is used as the starting material, the lower alkanoic acid generated upon salt exchange reaction and ethanol as the solvent form an ester having a lower boiling point, which can be readily removed by spray drying.

In general the apparatus and condition for spray drying may be the same as those used for producing amorphous medicines or food. In the case of a nonaqueous solvent, a spray drying apparatus which can recover the solvent is preferred. The feed stock solution may be used in any concentrations so long as it is not saturated. When a good flowability is required, a dilute feed stock solution is preferred.

The particle size of the resulting amorphous powder can be controlled by changing the concentration of the feed stock solution, the size of the spray nozzle or the feed rate. The feed rate depends on the capacity of the apparatus but is preferred to be controlled according to the particle size or the dryness of the resulting powder. The inlet temperature of the chamber of the apparatus is preferably set within a range of from 30 to 200°C, depending on the boiling point of the solvent and the dryness, more preferably within a range of from 40 to 160°C. As the powder obtained by spray drying is usually charged with static electricity, it is preferred to earth the powder receiver to eliminate static electricity.

In the above mentioned steps, by forming and spray drying the hydrochloride virtually in the absence of water, it is possible to obtain the hydrochloride in the anhydrous form. The amorphous paroxetine hydrochloride of the present invention is preferably an anhydride obtained virtually in the absence of water.

The amorphous paroxetine hydrochloride of the present invention is a powder which substantially contains no crystals. Figure 1 is the X-ray powder diffraction pattern of the amorphous paroxetine hydrochloride obtained by spray drying. It clearly indicates the halo effect, and the quite low intensity suggests an amorphous pattern. In contrast, the X-ray powder diffraction pattern of crystals of the hemihydrate paroxetine hydrochloride disclosed in Japanese Examined Patent Publication JP-B-6-47587 (Figure 1 in the publication) shows a clear peak.

Now, the present invention will be described in further detail with reference to Examples. However, it should be understood that the present invention is by no means restricted to such specific examples.

### EXAMPLE 1

1.51g of paroxetine was dissolved in 30 ml of ethanol, and 3 ml of a 22 wt% solution of hydrogen chloride in ethanol was added under cooling with ice. The resulting solution was left to stand at room temperature for an hour to obtain a solution of paroxetine hydrochloride in ethanol.

The solution of paroxetine hydrochloride in ethanol was spray dried by a spray drying machine for organic solvents (type: GS31, manufactured by Yamato Scientific Corporation) under the conditions of the nozzle size: 0.4 mm0̸, the feed rate: 11.7 g/min and the inlet temperature: 90°C, to obtain 1.2g of anhydrous amorphous paroxetine hydrochloride.

¹H NMR(400MHz,CDCl₃) δ 9.9(br,2H);7.20-7.23(m,2H); 6.98-7.02(m,2H);6.62(d,J=8.4Hz,1H);6.34(d,J=2.2Hz,1H); 6.12(dd,J=2.2,8.4Hz,1H);5.89(s,2H);3.73(br,1H);3.67 (br,1H);3.60(m,1H);3.48(m,1H);3.17(br,1H);3.05(br,1H); 2.92(br,1H);2.66(br,1H);2.40(br,1H);2.04(br,1H).

¹⁹F NMR(376MHz,CDCl₃,CFCl₃=0ppm) δ -115.4.

Figure 1 is the X-ray powder diffraction pattern of the amorphous paroxetine hydrochloride obtained by the following instrument.

### Instrument: Rotorflex RU-200, manufactured by Rigaku Denki Company Limited

### Measuring conditions:

Tube target: Cu, Tube voltage; 50 kV, Tube current; 200 mA,
Sampling width; 0.010°, Scanning rate; 0.500°/minute,
Scanning axis; 2θ/θ, Divergent slit; 1°, Scattering slit; 1°,
Receiving slit; 0.30 mm.

### EXAMPLE 2

13.9g of paroxetine was dissolved in 100 ml of ethanol, and 10 ml of a 22 wt% solution of hydrogen chloride in ethanol was added to obtain a solution of paroxetine hydrochloride in ethanol. The solution of paroxetine hydrochloride in ethanol was concentrated in vacuo, and 3.2g of the residue was dissolved in 100 ml of ethanol. This solution was spray dried with the same machine under the same conditions as in Example 1 to obtain 2.3g of anhydrous amorphous paroxetine hydrochloride.

¹H NMR(400MHz,DMSO-d₆) δ 8.80(br,2H);7.22-7.25(m,2H); 7.14-7.19(m,2H);6.74(d,J=8.4Hz,1H);6.49(d,J=2.4Hz,1H); 6.19(dd,J=8.4Hz,2.4Hz,1H);5.93(s,2H);3.59(m,1H);3.49 (m,2H);3.42(m,1H);2.97(m,2H);2.86(m,1H);2.67(br,1H);1.97 (m,1H);1.90(m,1H).

¹⁹F NMR(376MHz,DMSO-d₆,CFCl₃=0ppm)δ -116.01.

### EXAMPLE 3

9.8g of paroxetine acetate was dissolved in 80 ml of ethanol, and then 12 ml of a 22 wt% solution of hydrogen chloride in ethanol was added to obtain a solution of paroxetine hydrochloride in ethanol. 40 ml of the solution of paroxetine hydrochloride in ethanol was spray dried with the same machine under the same conditions as in Example 1 to obtain 3.5g of anhydrous amorphous paroxetine hydrochloride.

### EXAMPLE 4

40 ml of the solution of paroxetine hydrochloride in ethanol obtained in Example 3 was concentrated in vacuo, and the residue was dissolved in 60 ml of ethanol. This solution was spray dried with the same machine under the same conditions as in Example 1 to obtain 3.1g of anhydrous amorphous paroxetine hydrochloride.

### EXAMPLE 5

10.5g of paroxetine was dissolved in 950 ml of water, and 11 ml of 3N hydrochloric acid was added under cooling with ice to obtain an aqueous solution of paroxetine hydrochloride. The aqueous solution of paroxetine hydrochloride was spray dried by using a mini spray drier (type: GA32, manufactured by Yamato Scientific Corporation) under the conditions of the nozzle size: 0.4 mmø, the feed rate: 9.4 g/min and the inlet temperature: 160°C, to obtain 7.8g of hydrous amorphous paroxetine hydrochloride.

### EXAMPLE 6

1146g of paroxetine acetate was dissolved in 5.4L of ethanol, and then a solution of hydrogen chloride in ethanol (221g/1.6L) was added to obtain a solution of paroxetine hydrochloride in ethanol. This solution was concentrated in vacuo, and the residue was dissolved in 36L of ethanol.

The solution of paroxetine hydrochloride in ethanol was spray-dried by a spray drying machine for organic solvents (type: DA2SW-16, manufactured by Sakamoto Engineering CO., LTD.) under the conditions of the revolution of atomizer: 10,000 - 12,000 rpm, the feed rate: 3 - 4 L/hr and the inlet temperature: 110 - 130°C, the obtain 523.4g of anhydrous amorphous paroxetine hydrochloride.

It is possible to produce amorphous paroxetine hydrochloride which is useful as a medicine.

## Claims

1. A method of producing amorphous paroxetine hydrochloride, which comprises converting paroxetine or a lower alkanoic acid salt HAVING AT MOST 6 CARBON ATOMS thereof into paroxetine hydrochloride and then spray drying a solution of the hydrochloride.

2. The method according to Claim 1, wherein paroxetine or a lower alkanoic acid salt thereof is converted into paroxetine hydrochloride in water or a saturated alcohol.

3. The method according to Claim 1, wherein paroxetine or a lower alkanoic acid salt thereof is converted into paroxetine hydrochloride in a lower saturated alcohol.

4. The method according to Claim 1 or 3, wherein paroxetine or a lower alkanoic acid salt thereof is converted into paroxetine hydrochloride in a lower saturated alcohol, and the resulting solution of paroxetine hydrochloride in the lower saturated alcohol is spray dried.

5. The method according to any one of Claims 1, 3 and 4, wherein anhydrous amorphous paroxetine hydrochloride is produced in the absence of water.

## Patentansprüche

1. Verfahren zur Herstellung von amorphem Paroxetin-Hydrochlorid, umfassend die Umwandlung von Paroxetin oder einem Niederalkansäuresalz davon mit höchstens sechs Kohlenstoffatomen, zu Paroxetin-Hydrochlorid und anschließend Sprühtrocknen einer Lösung des Hydrochlorids.

2. Verfahren nach Anspruch 1, wobei Paroxetin oder ein Niederalkansäuresalz davon in Wasser oder einem gesättigten Alkohol zu Paroxetin-Hydrochlorid umgewandelt wird.

3. Verfahren nach Anspruch 1, wobei Paroxetin oder ein Niederalkansäuresalz davon in einem gesättigten Niederalkohol zu Paroxetin-Hydrochlorid umgewandelt wird.

4. Verfahren nach Anspruch 1 oder 3, wobei Paroxetin oder ein Niederalkansäuresalz davon in einem gesättigten Niederalkohol zu Paroxetin-Hydrochlorid umgewandelt wird und die erhaltene Lösung von Paroxetin-Hydrochlorid in dem gesättigten Niederalkohol sprühgetrocknet wird.

5. Verfahren nach einem der Ansprüche 1, 3 und 4, wobei das wasserfreie, amorphe Paroxetin-Hydrochlorid in Abwesenheit von Wasser hergestellt wird.

## Revendications

1. Procédé de production de chlorhydrate de paroxétine amorphe, qui comprend la conversion de paroxétine ou d'un sel de cette dernière avec un acide alcanoïque inférieur ayant au plus 6 atomes de carbone, en chlorhydrate de paroxétine, puis séchage par atomisation d'une solution du chlorhydrate.

2. Procédé selon la revendication 1, dans lequel la paroxétine ou un sel de cette dernière avec un acide alcanoïque inférieur est converti en chlorhydrate de paroxétine dans de l'eau ou un alcool saturé.

3. Procédé selon la revendication 1, dans lequel la paroxétine ou un sel de cette dernière avec un acide alcanoïque inférieur est converti en chlorhydrate de paroxétine dans un alcool saturé inférieur.

4. Procédé selon la revendication 1 ou 3, dans lequel la paroxétine ou un sel de cette dernière avec un acide alcanoïque inférieur est converti en chlorhydrate de paroxétine dans un alcool saturé inférieur, et la solution obtenue de chlorhydrate de paroxétine dans l'alcool saturé inférieur est séchée par atomisation.

5. Procédé selon l'une quelconque des revendications 1, 3 et 4, dans lequel du chlorhydrate de paroxétine amorphe anhydre est produit en l'absence d'eau.
